# FASCICULE DE BREVET EUROPEEN

(11) **EP 2 765 956 B1**
(45) Date de publication et mention de la délivrance du brevet: **23.03.2016**
(21) Numéro de dépôt: 12779125.9
(22) Date de dépôt: 12.10.2012
(51) Int. Cl.: A61F 2/44

(54) **IMPLANT INTERSOMATIQUE**
INTERSOMATISCHES IMPLANTAT
INTERSOMATIC IMPLANT

(30) Priorité: 14.10.2011 FR 1159298
(43) Date de publication de la demande: 20.08.2014
(73) Titulaire: Roussouly, Pierre, 69450 Saint Cyr au Mont d'Or (FR); Hilmi, Radwan, 69620 Bagnols (FR); Silvestre, Clément, 69340 Francheville (FR); Vallese, Pierre, 01001 Bourg En Bresse (FR); Arthroplastie Diffusion, 01800 Saint-Maurice-de-Gourdans (FR)
(72) Inventeur: ROUSSOULY, Jean-Charles, F-69100 Villeurbanne (FR); ROY, Christophe, F-26300 Chatuzange le Goubet (FR)
(74) Mandataire: Delorme, Nicolas
(86) Numéro de dépôt international: PCT/FR2012/052327
(87) Numéro de publication internationale: WO 2013/054057

(56) Documents cités:
- WO-A1-01/49219
- WO-A1-2004/024037
- WO-A1-2005/065596
- WO-A1-2010/021612
- US-A- 5 514 180
- US-A1- 2004 073 314

## Description

La présente invention a pour objet un implant intersomatique encore appelé implant intervertébral, destiné à être inséré dans l'espace intervertébral défini par deux vertèbres adjacentes de la colonne vertébrale, encore appelée rachis, dans le but de stabiliser lesdites vertèbres dans une position anatomique normale, après ablation du disque intervertébral défectueux. Un implant intersomatique est décrit dans le document US 2004/0073314.

Une telle opération de remplacement par un implant d'un disque intervertébral endommagé est appelée une arthrodèse intercorporéale : elle peut être nécessaire suite à divers défauts observés sur un disque intervertébral. Par exemple, le disque intervertébral peut avoir été tassé sur l'ensemble de sa surface et la hauteur initiale entre les deux vertèbres adjacentes a été perdue. Dans d'autres cas, le disque intervertébral peut avoir été tassé de façon asymétrique, dans une de ses régions latérales, antérieure et/ou postérieure. Le patient peut ainsi être atteint de scoliose. La colonne vertébrale étant le siège de structures neurologiques fondamentales, de tels phénomènes provoquent des douleurs chez les patients et il est nécessaire de procéder à l'ablation du disque intervertébral défectueux et de le remplacer par un implant.

Dans la présente demande, on entend, par région « antérieure » d'un disque intervertébral ou d'un implant intersomatique, la partie du disque ou de l'implant positionnée ou destinée à être positionnée en regard de l'abdomen du patient. Dans la présente demande, par « région antérieure » en général, on entend la région du patient située vers l'avant par rapport à la colonne vertébrale, autrement dit l'abdomen du patient. Dans la présente demande, on entend, par région « postérieure » d'un disque intervertébral ou d'un implant intersomatique, la partie du disque ou de l'implant positionnée ou destinée à être positionnée en regard du dos du patient. Dans la présente demande, par « région postérieure » en général, on entend la région du patient située vers l'arrière par rapport à la colonne vertébrale, autrement dit le dos du patient.

Les implants intervertébraux statiques destinés à l'arthrodèse, appelés communément « cages », sont généralement des cales rigides à hauteur fixe avec ou sans pente postérieure. Certaines de ces cages peuvent être creuses afin de pouvoir accueillir des matériaux tels que de l'os autogène, des phospho-calcites de synthèse ou des ostéo-inducteurs destinés à réaliser une fusion entre les plateaux des vertèbres adjacentes, encore appelée fusion secondaire. Une telle fusion secondaire permet de maintenir la correction de la déformation réalisée par l'implantation de l'implant intervertébral.

Certains implants existants peuvent être adaptés à l'espace créé lors de l'ablation du disque intervertébral avant leur implantation. Toutefois, seule leur pente antéropostérieure peut ainsi être modifiée : ces implants n'agissent donc que sur la hauteur antérieure de l'espace intervertébral pour tenter de corriger la lordose lombaire.

D'autres techniques chirurgicales préconisent l'emploi de deux cages de hauteurs différentes introduites par voie postérieure ou postéro-latérale pour tenter de régler l'obliquité du rachis mais l'introduction de ces cages nécessite d'importantes résections osseuses des facettes, des pédicules ou de l'arc postérieur vertébral des vertèbres concernées, ce qui peut aggraver l'instabilité de la colonne. Par ailleurs, la mise en place de telles cages comporte des risques de lésion des racines ou de la dure-mère.

Les implants ou cages existants sont généralement réalisés sous forme de blocs plus ou moins curvilignes et/ou convexes, pour tenter de suivre la concavité naturelle des plateaux vertébraux. Cette concavité est en fait de forme torique ovalisée avec un léger renflement central : la forme des implants existants ne permet pas leur adaptation correcte à la concavité des plateaux vertébraux, ni la stabilité nécessaire à la prise de la greffe osseuse lorsque cette dernière est souhaitée.

Il est, bien sûr, possible de modeler les plateaux vertébraux à la forme de l'implant pour créer un semblant de stabilité, mais ce creusement se fait au détriment de la fine couche d'os sous-chondral supportant le disque. Dès lors l'implant s'enfonce dans le spongieux du corps vertébral et la correction per-opératoire diminue ou disparaît.

Ainsi, les implants ou cages dits « PLIF », c'est-à-dire implantés par abord postérieur, sont délordosants et possèdent une petite surface de greffe sans atteinte de l'os sous chondral. Les implants ou cages dits « TLIF », c'est-à-dire implantés par abord postéro latéral, peuvent rendre la lordose, mais souvent au détriment de l'os sous chondral et toujours avec une petite surface de greffe. Enfin, les implants ou cages dits « ALIF », c'est-à-dire par abord antérieur, nécessitent une technique d'implantation compliquée (réclinement de l'axe aortocave) : l'implant est difficile à caler entre les deux vertèbres adjacentes.

La mauvaise adaptation et l'instabilité des implants existants sont des facteurs négatifs pour une bonne fusion osseuse, gage elle-même de la pérennité de la correction per-opératoire.

En outre, la nature statique des implants existants ne permet pas l'existence de micro mouvements pour accélérer et densifier la greffe osseuse secondaire.

La présente invention vise à remédier aux inconvénients cités ci-dessus.

La présente invention se rapporte à un implant intersomatique destiné à être inséré dans l'espace intervertébral défini par deux vertèbres voisines, comprenant un corps présentant au moins une face supérieure destinée à être placée en regard de la vertèbre supérieure, et une face inférieure, destinée à être placée en regard de la vertèbre inférieure, séparées l'une de l'autre par la hauteur de l'implant et sensiblement parallèles entre elles, les périphéries desdites face supérieure et inférieure étant reliées entre elles par une paroi périphérique sensiblement verticale, chacune desdites faces supérieure et inférieure présentant sensiblement la forme d'un triangle convexe aux angles arrondis, chaque face comprenant trois surfaces d'appui, destinées à être en contact avec le plateau de la vertèbre adjacente, lesdites surfaces d'appui se situant substantiellement au niveau des trois angles arrondis dudit triangle,
caractérisé en ce que
au moins une des faces supérieure et inférieure est munie de moyens de guidage de l'insertion de l'implant dans l'espace intervertébral selon une direction déterminée, lesdits moyens de guidage comprenant une pluralité de crêtes ménagées sur ladite face, lesdites crêtes étant dirigées selon un arc de cercle dont le centre serait situé dans la région antérieure externe à l'implant.

L'implant selon l'invention présente ainsi globalement la forme d'un trépied ou tripode destiné à s'appuyer à trois endroits différents du plateau vertébral des vertèbres adjacentes, en particulier au niveau de la concavité de ces plateaux. La présence de trois surfaces d'appui confère une excellente stabilité à l'implant. L'implant selon l'invention permet de restaurer ou corriger la hauteur, l'inclinaison sagittale et/ou l'alignement frontal de l'espace intervertébral.

Par ailleurs, l'implant selon l'invention ne nécessite pas de modifier, par exemple par creusement, la structure anatomique initiale des plateaux vertébraux et permet donc de respecter l'intégrité de l'os sous chondral. L'implant selon l'invention permet de réaliser une adaptation et une stabilité automatique et immédiate, sans artifices ni compromis de correction dans les trois plans, à savoir le plan frontal, le plan horizontal et le plan sagittal. De plus, la forme particulière de l'implant, à savoir globalement celle d'un trépied, est particulièrement adaptée pour une introduction sûre de l'implant par la voie d'abord rétropéritonéale.

Dans une forme de réalisation de l'invention, chaque surface d'appui de la face supérieure est reliée à la surface d'appui correspondante de la face inférieure par au moins une paroi sensiblement verticale formant un pilier d'appui. Par « sensiblement verticale », on entend, selon la présente demande, que la paroi peut être légèrement inclinée par rapport à la verticale. Ainsi, les piliers d'appui peuvent être verticaux ou légèrement inclinés par rapport à la verticale. Les piliers d'appui peuvent être parallèles entre eux, ou légèrement convergents ou divergents. La présence de tels piliers d'appui permet d'obtenir une stabilité immédiate de l'implant dans l'espace intervertébral. La présence de piliers d'appui renforce la résistance de l'implant qui doit, lorsqu'il est implanté, supporter la contrainte exercée sur lui à la fois par la vertèbre supérieure et par la vertèbre inférieure. Le contact entre les surfaces d'appui de l'implant et les parties concaves des plateaux vertébraux des deux vertèbres adjacentes est ainsi optimal.

Dans une forme de réalisation de l'invention, ledit corps est plein. Une telle forme de réalisation de l'implant selon l'invention permet de restaurer la hauteur de l'espace intervertébral, le parallélisme des plateaux des deux vertèbres adjacentes, une lordose lombaire ou l'alignement frontal, en fonction de la hauteur de chaque pilier d'appui, cette hauteur étant déterminée par le chirurgien en fonction du défaut à corriger.

Dans une autre forme de réalisation de l'invention, ledit corps est muni d'au moins un évidement traversant de ladite face supérieure à ladite face inférieure. Par exemple, ledit corps est muni de trois évidements, chaque évidement correspondant à un noyau central d'un pilier d'appui. Alternativement ou en combinaison, au moins une des faces supérieure et inférieure, et de préférence chaque face, est munie d'une ou plusieurs parties creusées, non traversantes. La présence de tels évidements et/ou parties creusées permet de remplir l'implant avec un matériau de comblement, tels que des greffons osseux, destiné à fusionner avec l'os des vertèbres adjacentes, en vue de fixer l'implant à ces vertèbres par fusion secondaire.

Dans une forme de réalisation de l'invention, ledit triangle est un triangle isocèle dont la base présente une longueur plus grande que celle des deux côtés égaux. Une telle forme de réalisation permet une adéquation optimale et donc un contact excellent entre d'une part les surfaces d'appui des piliers d'appui de l'implant selon l'invention et la concavité en forme de tore ovalisé du plateau vertébral de chaque vertèbre adjacente. En particulier, comme il apparaîtra de la suite de la description, la base du triangle isocèle est située dans la région antérieure de l'implant selon l'invention, et le sommet du triangle isocèle est situé dans la région postérieure de l'implant selon l'invention.

Par exemple, les évidements correspondant aux noyaux centraux des deux piliers d'appui situés au niveau des deux angles de même mesure du triangle isocèle sont reliés entre eux par un défaut de matière, l'ensemble desdits deux évidements et du défaut de matière formant ensemble un évidement continu en forme de haricot. Un tel évidement continu en forme de haricot correspond de façon idéale à la forme de la concavité des plateaux vertébraux des vertèbres adjacentes. La fusion secondaire est donc optimisée.

Par ailleurs, dans un tel cas, de préférence, les bords externes des deux piliers d'appui situés au niveau des deux angles de même mesure du triangle isocèle présentent une hauteur légèrement inférieure à celle des centres de ces piliers d'appui. La forme en haricot de l'évidement continu et la hauteur réduite des bords externes de ces piliers d'appui favorisent l'introduction de l'implant par un abord limité du disque intervertébral. En comparaison, les implants intersomatiques classiques plus massifs imposent une résection totale de l'annulus du disque (périphérie fibreuse) et par conséquent il est nécessaire de refouler totalement en arrière le muscle iliaque qui passe le long de la face latérale du rachis lombaire. Cette action peut être délétère pour les racines nerveuses qui passent à proximité.

Dans l'implant selon l'invention, au moins une des faces supérieure et inférieure est munie de moyens de guidage de l'insertion de l'implant dans l'espace intervertébral selon une direction déterminée, en particulier selon la direction antéro-latérale rétro-péritonéale. De tels moyens de guidage facilitent l'introduction de l'implant dans l'espace intervertébral, en particulier son insertion entre les deux plateaux vertébraux des vertèbres adjacentes, une telle insertion se faisant généralement à force. Ainsi, lesdits moyens de guidage comprennent une pluralité de crêtes ménagées sur ladite face, lesdites crêtes étant dirigées selon un arc de cercle dont le centre serait situé dans la région antérieure externe à l'implant. En particulier, les crêtes sont sensiblement parallèles entre elles. Ainsi, dans la position implantée de l'implant selon l'invention, le centre de l'arc de cercle sur lequel sont alignées lesdites crêtes est situé dans la région de l'abdomen du patient. Une telle forme de réalisation est particulièrement avantageuse pour une insertion de l'implant selon l'invention par une voie d'abord antéro-latérale rétro-péritonéale. De préférence, lesdits moyens de guidage, en particulier les crêtes en arc de cercle, sont présents sur la face supérieure et sur la face inférieure de l'implant. Ainsi, une fois l'implant inséré entre les deux plateaux vertébraux, la présence des moyens de guidage, et particulièrement des crêtes en arc de cercle, permet d'empêcher l'implant de bouger par rapport à ces deux plateaux et de le maintenir fixé fermement dans la position souhaitée par le chirurgien, et ce quelle que soit la voie d'abord utilisée pour insérer l'implant entre les deux vertèbres. De plus, la direction d'implantation en particulier antéro-latérale rétro-péritonéale, en alignement avec les moyens de guidage, à savoir les crêtes en arc de cercle, entraîne un cheminement concave en avant de l'implant lors de son insertion, l'empêchant ainsi de s'orienter vers l'arrière du corps vertébral où il risque d'endommager les structures nerveuses.

Dans une forme de réalisation de l'invention, tous les piliers d'appui présentent la même hauteur. Une telle forme de réalisation permet de reconstituer de façon simple et immédiate le parallélisme des plateaux vertébraux des vertèbres adjacentes.

Alternativement, au moins un des trois piliers d'appui présente une hauteur différente de la hauteur des deux autres piliers d'appui. Dans encore une autre forme de réalisation, chaque pilier d'appui a une hauteur différente. De telles formes de réalisation permettent de restaurer la lordose lombaire ou l'alignement frontal, comme expliqué plus en détails ci-dessous.

La présente invention ressortira plus clairement de la description qui suit et des dessins annexés dans lesquels :
La Figure 1 est une vue en perspective d'un implant selon l'invention,
La Figure 2 est une vue de dessus de l'implant de la Figure 1,
La Figure 3 est un schéma représentant les pilers d'appui sur l'implant de la Figure 2,
La Figure 4 est une vue de face de l'implant de la Figure 1,
La Figure 5 est une vue en perspective de côté de l'implant de la Figure 1,
La Figure 6 est une vue de dessus d'une autre forme de réalisation d'un implant selon l'invention,
La Figure 7 est une vue en perspective de l'implant de la Figure 6,
La Figure 8 est une vue schématique de la voie chirurgicale d'insertion de l'implant selon l'invention,
La Figure 9 est une vue schématique de dessus de l'implant selon l'invention une fois inséré entre deux vertèbres,
La Figure 10 est une vue de côté de l'implant selon l'invention, une fois inséré entre deux vertèbres.

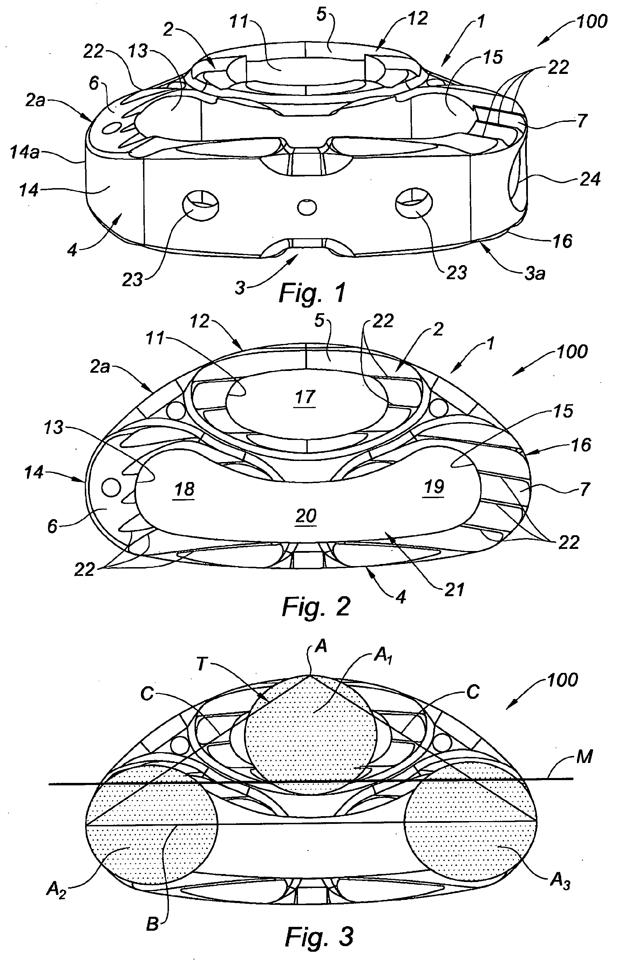

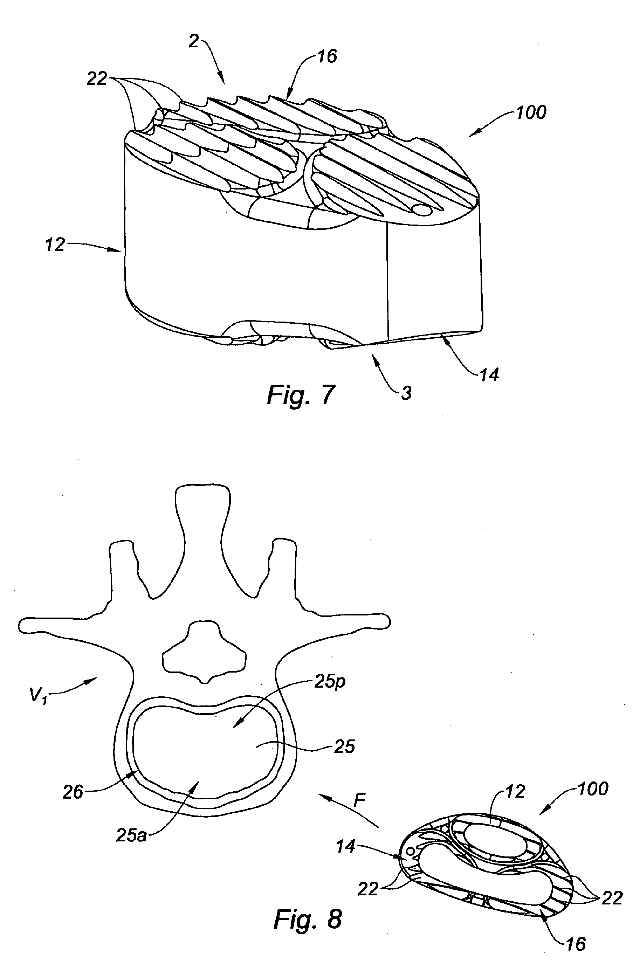

En référence à la Figure 1 est représenté en perspective un implant 100 intersomatique ou intervertébral selon l'invention. Comme il apparaîtra de la description ci-dessous, en particulier en référence aux figures 8-10, l'implant 100 est destiné à être inséré dans l'espace intervertébral défini par deux vertèbres voisines, ces vertèbres pouvant être deux vertèbres lombaires ou la vertèbre lombaire L5 et le plateau sacré, ou encore deux vertèbres cervicales.

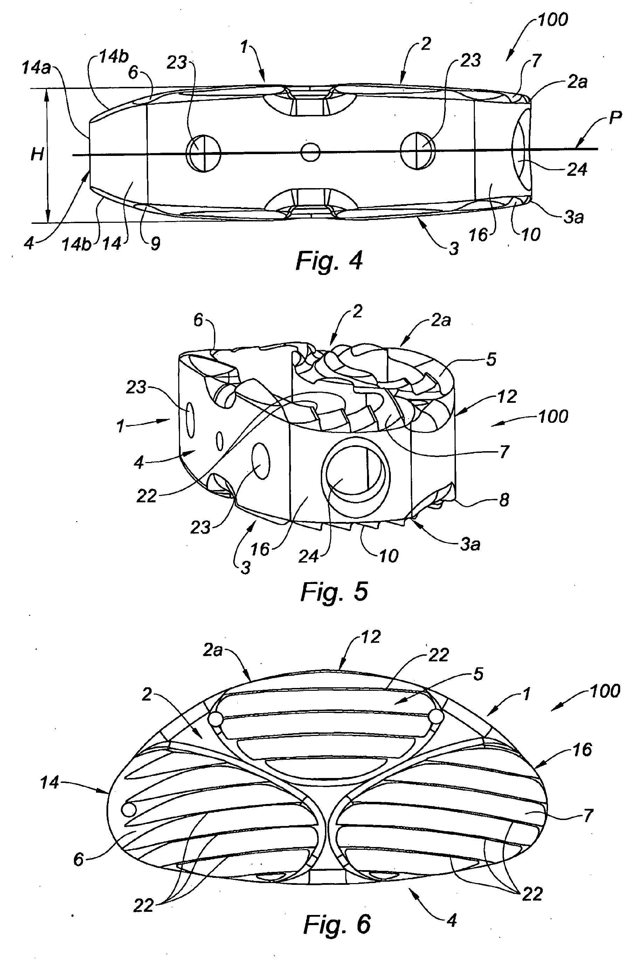

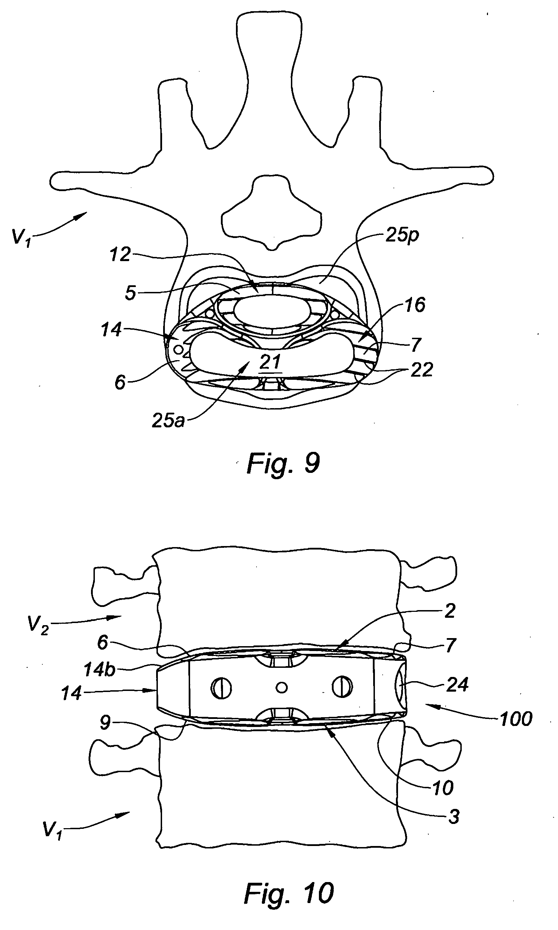

L'implant 100 comporte un corps 1 présentant une face supérieure 2, destinée à être placée en regard de la vertèbre supérieure (cf. Figure 10) lorsque l'implant est implanté, et une face inférieure 3 destinée à être placée en regard de la vertèbre inférieure (cf. Figure 10) lorsque l'implant est implanté, la face supérieure et la face inférieure étant séparées l'une de l'autre par la hauteur H (voir Figure 4) de l'implant 100. Le matériau formant le corps 1 peut être tout matériau biocompatible. Par exemple, le matériau formant le corps 1 de l'implant 100 est un matériau autorisant une légère déformation, de préférence élastique, de la forme de l'implant 100: une telle forme de réalisation permet, lorsque l'implant 100 contient des greffons osseux comme décrit ci-après, la dynamisation de la greffe lors des mouvements du rachis, afin d'accélérer la fusion et d'améliorer la qualité de cette dernière. De préférence, ce matériau est choisi parmi le titane, l'acier inoxydable, des polymères tels que le polyétheréthercétone, le polyéthercétonecétone et leurs mélanges. Par exemple, l'acier inoxydable peut être revêtu de titane.

Comme il apparaît de la Figure 4, les faces supérieure 2 et inférieure 3 sont sensiblement parallèles entre elles, c'est-à-dire qu'elles sont contenues dans des plans sensiblement parallèles entre eux. La face supérieure 2 présente une périphérie 2a et la face inférieure 3 présente une périphérie 3a. En référence aux figures 1, 4 et 5, les périphéries 2a et 3a des faces supérieure 2 et inférieure 3 sont reliées entre elles par une paroi périphérique 4, sensiblement verticale sur l'exemple représenté. Par « sensiblement verticale », on entend au sens de la présente demande que la paroi périphérique peut être verticale ou sensiblement inclinée par rapport à la verticale, ladite paroi reliant les périphéries respectives des faces supérieure et inférieure de l'implant sur l'ensemble de la hauteur de l'implant. L'implant 100 étant destiné à prendre la place d'un disque intervertébral défectueux, il présente une hauteur, désignée par H sur la Figure 4, sensiblement égale à celle d'un tel disque intervertébral dans son état naturel non endommagé.

Comme il ressort des Figures 1 à 3, chaque face (2 ; 3) de l'implant 100 présente sensiblement la forme d'un triangle convexe dont les angles seraient arrondis ; par ailleurs, sur l'exemple représenté, l'implant 100 est symétrique par rapport à un plan horizontal P passant par la moitié de la hauteur H, comme montré sur la Figure 4.

Le triangle définissant la forme convexe de chaque face peut être quelconque, équilatéral ou encore isocèle. De préférence, ce triangle est isocèle. De préférence, le triangle isocèle présente une base de longueur plus grande que celle des deux côtés égaux. Le triangle isocèle peut ainsi être qualifié d'aplati.

Le schéma de la Figure 3 aide à visualiser un tel triangle, qui a été représenté et qui est désigné par la lettre T. Le triangle T, définissant la forme de la face supérieure 2 de l'implant 100, est un triangle isocèle présentant un sommet principal A, une base B et deux côtés C égaux, i.e. de même longueur. Comme il apparaît de la Figure 3, la longueur de la base B est supérieure à celle de chacun des côtés C, conférant ainsi au triangle T, et à la face supérieure 2, un aspect aplati. La face inférieure 3 est identique à la face supérieure 2 par symétrie par rapport au plan P (cf. Figure 4).

Sur la Figure 3, les angles arrondis du triangle T ont été schématisés par des cercles A1, A2 et A3 : ces cercles A1-A3 correspondent à des surfaces d'appui 5, 6 et 7 présentes sur la face supérieure 2 de l'implant 100 comme montré sur la Figure 2. A ces trois surfaces d'appui, correspondent sur la face inférieure 3 respectivement trois surfaces d'appui 8, 9 et 10 (voir Figures 4 et 5) symétriques par rapport au plan P (voir Figure 4) : les surfaces d'appui (5-10) sont destinées à en être en contact avec le plateau de la vertèbre adjacente lorsque l'implant 100 est implanté (voir Figure 10), les surfaces d'appui 5-7 de la face supérieure 2 étant destinées à être en contact avec le plateau de la vertèbre supérieure, et les surfaces d'appui 8-10 de la face inférieure étant destinées à être en contact avec le plateau de la vertèbre inférieure. Comme il apparaît des Figures 1 et 7, les surfaces d'appui (5, 6, 7, 8, 9, 10) des deux faces (2, 3) supérieure et inférieure de l'implant 100 présentent une légère convexité adaptée à la concavité naturelle des plateaux vertébraux des vertèbres adjacentes.

Par exemple, chaque surface d'appui peut avoir une forme convexe, par exemple selon une partie de sphère ayant un rayon allant de 4 à 29 mm, de préférence égal au rayon de la concavité du plateau vertébral concerné. Par exemple, la surface d'une surface d'appui peut aller de 16 à 175 mm².

Comme il apparaîtra plus tard dans la description, la présence de trois surfaces d'appui sur les faces de l'implant 100 destinées à être en contact avec les deux vertèbres adjacentes permet de réaliser une adaptation et une stabilité automatique et immédiate, sans artifices ni compromis de correction dans les trois plans, à savoir à savoir le plan frontal, le plan horizontal et le plan sagittal.

En particulier, sur la Figure 3 a été représenté le plan médian M du rachis, lorsque l'implant 100 est implanté entre deux vertèbres. Comme il apparaît de cette Figure, les trois surfaces d'appui de l'implant, représentées par les cercles A1-A3 sont positionnées de telle façon que leurs forces sont réparties de façon optimale de part et d'autre du plan M de sorte que l'on obtient une stabilité systématique de l'implant, quelle que soit l'état de surface du plateau vertébral : on limite ainsi les risques de glissement de l'implant par rapport aux plateaux vertébraux des vertèbres adjacentes.

Dans l'exemple représenté sur les Figures 1-5, chaque surface d'appui (5, 6, 7) de la face supérieure 2 est reliée à la surface d'appui correspondante (8, 9, 10) de la face inférieure 3 par au moins une paroi verticale formant un pilier d'appui : ainsi, la surface d'appui 5 de la face supérieure 2 correspondant au sommet A du triangle T est reliée à la surface d'appui correspondante 8 (voir figure 5) de la face inférieure 3 par une paroi sensiblement verticale 11 formant un pilier d'appui 12. Les surfaces d'appui 6 et 7 de la face supérieure 2 et correspondant aux deux autres angles du triangle T sont reliées aux surfaces d'appui correspondantes 9 et 10 (voir Figure 4) de la face inférieure 3 respectivement par des parois sensiblement verticales 13 et 15 formant respectivement les piliers d'appui 14 et 16.

La présence de piliers d'appui renforce la résistance de l'implant 100 qui doit, lorsqu'il est implanté, supporter la contrainte exercée sur lui à la fois par la vertèbre supérieure et par la vertèbre inférieure. Le contact entre les surfaces d'appui de l'implant et des plateaux vertébraux des deux vertèbres adjacentes est ainsi optimal. En particulier, comme il apparaîtra à la figure 9, une telle forme de réalisation où le triangle définissant la forme des faces de l'implant est isocèle permet une adéquation optimale et donc un contact excellent entre d'une part les surfaces d'appui des piliers d'appui de l'implant selon l'invention et la concavité en forme de tore ovalisé du plateau vertébral de chaque vertèbre adjacente.

Dans l'exemple représenté sur les Figures 1-5, l'implant 100 est muni d'évidements traversant de la face supérieure 2 à la face inférieure 3. Dans l'exemple représenté, ces évidements sont au nombre de trois et ils correspondent globalement à un noyau central de chaque pilier d'appui. Ainsi, le pilier d'appui 12 est traversé par un évidement central 17. Les piliers d'appui 14 et 16 sont chacun traversés par un évidement central 18 et 19, ces deux évidements étant reliés entre eux par un défaut de matière 20 de façon à former un évidement continu 21 en forme de haricot (voir Figure 2).

Alternativement ou en combinaison, dans une forme de réalisation non représentée, au moins une des faces supérieure et inférieure, et de préférence chaque face, est munie de parties creusées, non traversantes.

La présence de tels évidements et/ou parties creusées permet de remplir l'implant 100 avec un matériau de comblement, tels que des greffons osseux, destiné à fusionner avec l'os des vertèbres adjacentes, en vue de fixer l'implant à ces vertèbres.

Dans la forme de réalisation de l'implant 100 selon l'invention représentée aux Figures 6 et 7, pour lesquelles les références désignant les mêmes éléments qu'aux Figures 1-5 ont été conservées, le corps 1 est plein et est donc dépourvu d'évidements. Un tel implant permet de restaurer la hauteur nécessaire entre deux vertèbres. Par ailleurs, lorsqu'une fusion secondaire n'est pas nécessaire, une telle forme de réalisation permet de reconstituer de façon simple et immédiate le parallélisme des plateaux, la lordose lombaire ou encore l'alignement frontal, en fonction des hauteurs respectives des piliers d'appui, comme expliqué ci-après.

Sur les exemples représentés aux figures 1-10, les trois piliers d'appui 12, 14, et 16 ont sensiblement la même hauteur. Une telle forme de réalisation permet de reconstituer de façon simple et immédiate le parallélisme des plateaux vertébraux des vertèbres adjacentes. Toujours sur les exemples représentés aux Figures 1-10, les bords externes des deux piliers d'appui (14, 16) situés au niveau des deux angles de même mesure du triangle isocèle présentent une hauteur légèrement inférieure à celle des centres de ces piliers d'appui : ainsi, comme il apparaît de la Figure 4, les faces supérieure et inférieure 14b du pilier d'appui 14 sont inclinées.

Dans d'autres formes de réalisation non représentées, au moins un pilier d'appui présente une hauteur différente de la hauteur des deux autres piliers d'appui. Dans la présente description, on désigne par pilier postérieur, destiné à être placé au niveau de la région postérieure des plateaux vertébraux des vertèbres adjacentes, le pilier d'appui 12, et par piliers antérieurs, destinés à être positionnés au niveau de la région antérieure des plateaux vertébraux des vertèbres adjacentes, les piliers d'appuis 14 et 16. Ainsi, le pilier postérieur 12 est situé dans la région postérieure de l'implant 100, et les piliers antérieurs (14, 16) sont situés dans la région antérieure de l'implant 100. Par exemple, lorsque le pilier postérieur 12 a une hauteur plus faible que la hauteur des deux piliers antérieurs 14 et 16, on peut reconstituer de façon simple et immédiate une lordose lombaire. Un autre exemple est celui où l'un des piliers antérieurs (14, 16) présente une hauteur plus importante que celle de l'autre pilier antérieur (16, 14), avec par exemple le pilier postérieur ayant une hauteur dont la valeur comprise entre celles des deux piliers antérieurs : dans un tel cas, on peut reconstituer l'alignement frontal pour corriger par exemple une scoliose ou compenser une vertèbre cunéiforme.

Dans d'autres formes de réalisation non représentées, chaque pilier d'appui a une hauteur différente. De telles formes de réalisation peuvent être utiles dans le cadre de déformations particulières.

Sur les exemples de réalisation de l'implant 100 représentés aux Figures 1 à 10, la face supérieure 2 est munie d'une pluralité de crêtes 22 : comme il apparaît sur le schéma de la Figure 3, ces crêtes 22 sont sensiblement parallèles entre elles et sont courbées selon un arc de cercle dont le centre serait situé dans la région antérieure externe à l'implant, autrement dit la région de l'abdomen lorsque l'implant 100 est implanté dans un patient, et dont le rayon varierait de 5 mm à 500 mm. Comme il apparaîtra de la description des Figures 8-10, la direction curviligne de ces crêtes correspond à la direction d'insertion de l'implant 100 lors de son implantation entre deux vertèbres voisines par une voie d'abord oblique antéro-latérale rétro-péritonéale : les crêtes forment ainsi des moyens de guidage de l'implant 100 lors de son insertion à force entre les deux vertèbres. Les crêtes 22, en entraînant un cheminement concave en avant de l'implant 100 lors de son insertion par voie d'abord oblique antéro-latérale rétro-péritonéale (voir Figure 8), protègent les structures nerveuses environnantes en empêchant l'implant de s'orienter vers l'arrière de la colonne vertébrale. Par ailleurs, comme il apparaît de la Figure 5, la face inférieure 3 de l'implant 100 est également munie de crêtes 22 présentant la même direction que les crêtes 22 de la face supérieure 2.

Comme montré sur la Figure 1, l'implant 100 peut en outre comprendre des orifices latéraux 23 : de tels orifices peuvent par exemple être utiles pour accueillir un outil dans le cas où l'ablation de l'implant s'avère nécessaire.

Sur l'exemple de réalisation montré aux figures 1-5, l'implant 100 est en outre muni d'un orifice latéral 24 situé au niveau de son pilier d'appui 16 : cet orifice latéral 24 est destiné à accueillir l'outil de mise en place de l'implant 100 lors de son insertion à force entre deux vertèbres. Dans une forme de réalisation non représentée, il est également possible de venir fixer dans l'orifice latéral 24 une plaque d'ostéosynthèse additive avec une vis permettant de fixer l'implant et les deux vertèbres adjacentes via un vissage dans chacune des vertèbres. Par ailleurs, comme il apparaît des Figures 1 et 4, la paroi verticale externe 14a du pilier d'appui 14 présente une hauteur légèrement inférieure à la hauteur H de l'implant 100 et les faces supérieure et inférieure 14b du pilier d'appui 14 sont inclinées. En effet, l'implant 100 étant destiné à être inséré à force entre deux vertèbres par le pilier d'appui 14, les faces 14b inclinées du pilier d'appui 14 sont ainsi atraumatiques et elles facilitent l'introduction de l'implant 100 dans l'espace intervertébral.

En référence aux Figures 8-10 va maintenant être décrite la technique chirurgicale d'insertion de l'implant 100 selon l'invention entre deux vertèbres dont le disque intervertébral, défectueux, doit être remplacé.

Sur la Figure 8, est représentée une vertèbre V1 vue de dessus avec son plateau vertébral 25. La région antérieure 25a du plateau vertébral est celle positionnée en regard de l'abdomen du patient (non représenté) alors que la région postérieure 25p du plateau vertébral 25 est positionnée vers le dos du patient. Ces deux régions 25a et 25p présentent une concavité disposée selon un tore ovalisé 26 représenté schématiquement sur la Figure 8. L'implant 100 selon l'invention est destiné à être inséré entre la vertèbre V1, inférieure, et la vertèbre V2 (voir Figure 10), supérieure, selon la direction indiquée par la flèche F sur la Figure 8, autrement dit selon une voie d'abord oblique antéro-latérale rétro-péritonéale. Comme il apparaît sur la Figure 8, les crêtes 22 sont sensiblement parallèles à la direction d'insertion F et elles vont constituer des moyens de guidage afin d'insérer l'implant 100 entre les deux vertèbres V1 et V2, de sorte que le pilier postérieur 12 de l'implant 100 vienne se positionner au niveau de la concavité de la région postérieure 25p du plateau vertébral 25 et que les deux piliers antérieurs 14 et 16 de l'implant 100 viennent se positionner au niveau de la concavité de la région 25a du plateau vertébral 25 de la vertèbre V1 (idem pour V2), comme montré sur la Figure 9.

En référence aux Figures 8-10, l'insertion de l'implant 100 se fait en approchant en premier lieu le pilier d'appui 14 de l'espace intervertébral : comme vu plus haut, le fait que le pilier d'appui 14 présente des faces supérieure et inférieure 14b inclinées facilite l'insertion de l'implant 100 entre les deux vertèbres V1 et V2. L'implant 100 est inséré à force entre les deux vertèbres à l'aide d'un outil (non représenté) dont une extrémité est reçue dans l'orifice latéral 24 et en faisant légèrement pivoter l'implant 100, selon la direction des crêtes 22 afin de positionner l'implant 100 comme expliqué plus haut et comme montré sur la Figure 9.

En référence à la Figure 10, l'implant 100 est maintenant implanté dans l'espace intervertébral défini par les deux vertèbres adjacentes V1 et V2. Grâce en particulier aux crêtes 22, l'implant 100 est fermement maintenu dans la position voulue par le chirurgien : les crêtes en arc de cercle 22 permettent un tel maintien en position, quelle que soit la voie d'abord qui a été utilisée pour mettre l'implant 100 en place. Comme il apparaît sur cette Figure, les crêtes 22 sont parallèles à un arc de cercle dont le centre est situé dans la région antérieure du patient, au-delà de l'implant 100, autrement dit dans la région de l'abdomen (non représenté) du patient, dans le plan médian du corps du patient.

Un tel implant selon l'invention permet de restaurer la hauteur de l'espace intervertébral, le parallélisme des plateaux des deux vertèbres adjacentes, une lordose lombaire ou l'alignement frontal, en fonction de la hauteur de chaque pilier d'appui, cette hauteur étant déterminée par le chirurgien en fonction du défaut à corriger.

Lorsque l'implant 100 comprend des greffons osseux, ces derniers vont fusionner avec les plateaux vertébraux des vertèbres V1 et V2 afin de pérenniser la correction de la déformation réalisée par l'implantation de l'implant selon l'invention.

## Revendications

1. Implant intersomatique (100) destiné à être inséré dans l'espace intervertébral défini par deux vertèbres voisines (V1, V2), comprenant un corps (1) présentant au moins une face supérieure (2) destinée à être placée en regard de la vertèbre supérieure, et une face inférieure (3), destinée à être placée en regard de la vertèbre inférieure, séparées l'une de l'autre par la hauteur de l'implant et sensiblement parallèles entre elles, les périphéries (2a, 3a) desdites face supérieure et inférieure étant reliées entre elles par une paroi périphérique (4) sensiblement verticale, chacune desdites faces supérieure et inférieure présentant sensiblement la forme d'un triangle convexe aux angles arrondis, chaque face comprenant trois surfaces d'appui (5, 6, 7, 8, 9, 10), destinées à être en contact avec le plateau de la vertèbre adjacente, lesdites surfaces d'appui se situant substantiellement au niveau des trois angles arrondis dudit triangle,
**caractérisé en ce que**,
au moins une des faces supérieure et inférieure (2, 3) est munie de moyens de guidage (22) de l'insertion de l'implant dans l'espace intervertébral selon une direction déterminée, lesdits moyens de guidage comprenant une pluralité de crêtes (22) ménagées sur ladite face (2, 3), lesdites crêtes étant dirigées selon un arc de cercle dont le centre serait situé dans la région antérieure externe à l'implant.

2. Implant (100) selon la revendication 1, **caractérisé en ce que** chaque surface d'appui (5, 6, 7) de la face supérieure (2) est reliée à la surface d'appui (8, 9, 10) correspondante de la face inférieure (3) par au moins une paroi sensiblement verticale (11, 13, 15) formant un pilier d'appui (12, 14, 16).

3. Implant (100) selon la revendication 1 ou 2, **caractérisé en ce que** ledit corps (1) est plein.

4. Implant (100) selon la revendication 1 ou 2, **caractérisé en ce que** ledit corps (1) est muni d'au moins un évidement (17, 18, 19, 20, 21) traversant de ladite face supérieure (2) à ladite face inférieure (3).

5. Implant (100) selon les revendications 2 et 4, **caractérisé en ce que** ledit corps (1) est muni de trois évidements (17, 18, 19), chaque évidement correspondant à un noyau central d'un pilier d'appui (14, 14, 16).

6. Implant (100) selon l'une quelconque des revendications 1 à 5, **caractérisé en ce que** ledit triangle est un triangle isocèle dont la base présente une longueur plus grande que celle des deux côtés égaux.

7. Implant (100) selon les revendications 5 et 6, **caractérisé en ce que** les évidements (18, 19) correspondant aux noyaux centraux des deux piliers d'appui (14, 16) situés au niveau des deux angles de même mesure du triangle isocèle sont reliés entre eux par un défaut de matière (20), l'ensemble desdits deux évidements (18, 19) et du défaut de matière (20) formant ensemble un évidement continu (21) en forme de haricot.

8. Implant (100) selon la revendication 7, **caractérisé en ce que** les bords externes des deux piliers d'appui situés au niveau des deux angles de même mesure du triangle isocèle présentent une hauteur légèrement inférieure à celle des centres de ces piliers d'appui.

9. Implant (100) selon l'une quelconque des revendications 1 à 8, **caractérisé en ce que** lesdites crêtes (22) sont présentes sur la face supérieure (2) et sur la face inférieure (3) de l'implant.

10. Implant (100) selon l'une quelconque des revendications 2 à 9, **caractérisé en ce que** tous les piliers d'appui (12, 14, 16) présentent la même hauteur.

11. Implant (100) selon l'une quelconque des revendications 2 à 9, **caractérisé en ce qu'**au moins un des trois piliers d'appui présente une hauteur différente de la hauteur des deux autres piliers d'appui.

12. Implant (100) selon l'une quelconque des revendications 2 à 9, **caractérisé en ce que** chaque pilier d'appui a une hauteur différente.

## Patentansprüche

1. Intersomatisches Implantat (100), das dazu vorgesehen ist, in den Zwischenwirbelraum eingeführt zu werden, der von zwei benachbarten Wirbeln (V1, V2) definiert wird, wobei das Implantat einen Körper (1) umfasst, der mindestens eine obere Fläche (2), die dazu vorgesehen ist, gegenüber dem oberen Wirbel platziert zu werden, und eine untere Fläche (3), die dazu vorgesehen ist, gegenüber dem unteren Wirbel platziert zu werden, aufweist, die durch die Höhe des Implantats voneinander getrennt sind und im Wesentlichen zueinander parallel sind, wobei die Umfänge (2a, 3a) der besagten oberen Fläche und der besagten unteren Fläche durch eine im Wesentlichen vertikale Umfangswand (4) miteinander verbunden sind, wobei jede der besagten oberen Fläche und der besagten unteren Fläche im Wesentlichen die Form eines konvexen Dreiecks mit abgerundeten Ecken aufweist, wobei jede Fläche drei Auflageflächen (5, 6, 7, 8, 9, 10) umfasst, die dazu vorgesehen sind, mit der Platte des benachbarten Wirbels in Kontakt zu stehen, wobei die besagten Auflageflächen sich im Wesentlichen auf Höhe von drei abgerundeten Ecken des besagten Dreiecks befinden,
**dadurch gekennzeichnet, dass**
mindestens eine der oberen Fläche und der unteren Fläche (2, 3) mit Führungsmitteln (22) zum Einführen des Implantats in den Zwischenwirbelraum gemäß einer bestimmten Richtung versehen ist, wobei die besagten Führungsmittel mehrere Grate (22) umfassen, die auf der besagten Fläche (2, 3) angeordnet sind, wobei die besagten Grate gemäß einem Kreisbogen geleitet sind, dessen Mitte sich in der vorderen Region extern zu dem Implantat befinden würde.

2. Implantat (100) nach Anspruch 1, **dadurch gekennzeichnet, dass** jede Auflagefläche (5, 6, 7) der oberen Fläche (2) mit der Auflagefläche (8, 9, 10), die der unteren Fläche (3) entspricht, durch mindestens eine im Wesentlichen vertikale Wand (11, 13, 15) verbunden ist, wodurch eine Auflagesäule (12, 14, 16) gebildet wird.

3. Implantat (100) nach Anspruch 1 oder 2, **dadurch gekennzeichnet, dass** der besagte Körper (1) massiv ist.

4. Implantat (100) nach Anspruch 1 oder 2, **dadurch gekennzeichnet, dass** der besagte Körper (1) mit mindestens einer Aussparung (17, 18, 19, 20, 21) versehen ist, die durch die besagte obere Fläche (2) zu der besagten unteren Fläche (3) verläuft.

5. Implantat (100) nach den Ansprüchen 2 und 4, **dadurch gekennzeichnet, dass** der besagte Körper (1) mit drei Aussparungen (17, 18, 19) versehen ist, wobei jede Aussparung einem mittleren Kern einer Auflagesäule (14, 14, 16) entspricht.

6. Implantat (100) nach einem der Ansprüche 1 bis 5, **dadurch gekennzeichnet, dass** das besagte Dreieck ein gleichschenkeliges Dreieck ist, dessen Basis eine Länge aufweist, die größer als die der gleichen Seiten ist.

7. Implantat (100) nach den Ansprüchen 5 und 6, **dadurch gekennzeichnet, dass** die Aussparungen (18, 19), die mittleren Kernen von zwei Auflagesäulen (14, 16) entsprechen, die sich auf Höhe von zwei Winkeln mit demselben Maß des gleichschenkeligen Dreiecks befinden, durch einen Materialfehler (20) miteinander verbunden sind, wobei die Einheit der besagten zwei Aussparungen (18, 19) und das Materialfehlers (20) zusammen eine durchgehende Aussparung (21) in Nierenform bilden.

8. Implantat (100) nach Anspruch 7, **dadurch gekennzeichnet, dass** die Außenränder von zwei Auflagesäulen, die sich auf Höhe von zwei Winkeln mit demselben Maß des gleichschenkeligen Dreiecks befinden, eine Höhe aufweisen, die geringfügig kleiner als die von Mitten dieser Auflageflächen ist.

9. Implantat (100) nach einem der Ansprüche 1 bis 8, **dadurch gekennzeichnet, dass** die besagten Grate (22) auf der oberen Fläche (2) und auf der unteren Fläche (3) des Implantats vorliegen.

10. Implantat (100) nach einem der Ansprüche 2 bis 9, **dadurch gekennzeichnet, dass** alle Auflagesäulen (12, 14, 16) dieselbe Höhe aufweisen.

11. Implantat (100) nach einem der Ansprüche 2 bis 9, **dadurch gekennzeichnet, dass** mindestens eine von drei Auflagesäulen eine Höhe aufweist, die sich von der Höhe der anderen beiden Auflagesäulen unterscheidet.

12. Implantat (100) nach einem der Ansprüche 2 bis 9, **dadurch gekennzeichnet, dass** jede Auflagesäule eine andere Höhe aufweist.

## Claims

1. An intersomatic implant (100) intended to be inserted into the intervertebral space defined by two neighboring vertebrae (V1, V2), comprising a body (1) having at least one upper face (2) intended to be placed opposite to the upper vertebra, and one lower face (3), intended to be placed opposite to the lower vertebra, separated from each other by the height of the implant and substantially parallel to each other, the peripheries (2a, 3a) of said upper and lower faces being interconnected by a peripheral wall (4) substantially vertical, each of said upper and lower faces having substantially the shape of a convex triangle with rounded corners, each face comprising three bearing surfaces (5, 6 , 7, 8, 9, 10), intended to be in contact with the endplate of the adjacent vertebra, said bearing surfaces being located substantially at the three rounded corners of said triangle,
**characterized in that**,
at least one of the upper and the lower faces (2, 3) is provided with means (22) for guiding the insertion of the implant into the intervertebral space along a predetermined direction, said guide means comprising a plurality of ridges (22) arranged on said face (2, 3), said ridges being directed along an arc of circle whose center would be located in the anterior area external to the implant.

2. The implant (100) according to claim 1, **characterized in that** each bearing surface (5, 6, 7) of the upper face (2) is connected to the corresponding bearing surface (8, 9, 10) of the lower face (3) by at least one substantially vertical wall (11, 13, 15) forming a bearing pillar (12, 14, 16).

3. The implant (100) according to claim 1 or 2, **characterized in that** said body (1) is full.

4. The implant (100) according to claim 1 or 2, **characterized in that** said body (1) is provided with at least one through-recess (17, 18, 19, 20, 21) from said upper face (2) to said lower face (3).

5. The implant (100) according to claims 2 and 4, **characterized in that** said body (1) is provided with three recesses (17,18,19), each recess corresponding to a central core of a bearing pillar (14, 14,16).

6. The implant (100) according to any one of claims 1 to 5, **characterized in that** said triangle is an isosceles triangle whose base has a length greater than that of the two equal sides.

7. The implant (100) according to claims 5 and 6, **characterized in that** the recesses (18, 19) corresponding to the central cores of the two bearing pillars (14, 16) located at the two corners with the same measure of the isosceles triangle are interconnected by a defect of material (20), the assembly of said two recesses (18, 19) and the defect of material (20) forming together a bean-shaped continuous recess (21).

8. The implant (100) according to claim 7, **characterized in that** the external edges of the two bearing pillars located at the two corners of the same measure of the isosceles triangle have a height slightly lower than that of the centers of these bearing pillars.

9. The implant (100) according to any one of claims 1 to 8, **characterized in that** said ridges (22) are present on the upper face (2) and on the lower face (3) of the implant.

10. The implant (100) according to any one of claims 2 to 9, **characterized in that** all the bearing pillars (12, 14, 16) have the same height.

11. The implant (100) according to any one of claims 2 to 9, **characterized in that** at least one of the three bearing pillars has a height different from the height of the other two bearing pillars.

12. The implant (100) according to any one of claims 2 to 9, **characterized in that** each bearing pillar has a different height.
